# EUROPEAN PATENT APPLICATION

(11) **EP 0 614 989 A1**
(43) Date of publication of application: **14.09.1994**
(21) Application number: 94102334.3
(22) Date of filing: 16.02.1994
(51) Int. Cl.: C12Q 1/70, C12Q 1/68, G01N 33/50, C12N 15/10

(54) **A method for in vivo selection of ligand-binding proteins**

(30) Priority: 17.02.1993 EP 93102484
(71) Applicant: MORPHOSYS GESELLSCHAFT FÜR PROTEINOPTIMIERUNG mbH, D-80807 München (DE)
(72) Inventor: Krebber,Claus, CH 8006 Zurich (CH); Moroney,Simon Dr, D 80805 Munich (DE); Plückthun,Andreas Dr, CH 8006 Zurich (CH); Schneider,Christian Dr, D 51429 Bergisch Gladbach (DE)
(74) Representative: VOSSIUS & PARTNER

(57) **Abstract**

The application discloses a method, and kit, which enables the selection of proteins or other oligo- or polypeptides (collectively, ligand binding proteins, LBPs) which bind with high affinity to a target ligand or receptor. The method relies on the display of the LBP of interest on the surface of replicable genetic packages (RGPs) which are modified so as to be non-infective. Infectivity is restored to those RGPs displaying LBPs with high affinity for a target ligand or receptor by an infectivity mediating complex. The infectivity mediating complex comprises the ligand or receptor covalently linked to a polypeptide which brings about interaction between the RGP and a host cell. It is envisaged that the LBP of interest will belong to a genetically diverse collection of similar substances. The method therefore allows selection of substances with high affinity for target, from within a large collection of variants.

## Description

### Field of the Invention

The present invention relates to a method for the selection of proteins or other oligo-or polypeptides which bind with high affinity to a target ligand or receptor. The invention relates in particular, but not exclusively, to a system which can be used to select among a large population of variants for antibodies having high affinity for a target antigen.

### Background to the Invention

The advent of methods for the production of functional antibody fragments in recombinant bacteria (1,2) has opened the way to genetic manipulation of antibody genes. As a result of this development, genetic engineering is increasingly being used to generate antibodies for particular applications. Examples of this methodology are antibody chimerization (3) and humanization (4). These processes produce antibodies better suited to human therapeutic applications than their typically murine progenitors, by altering parts of the antibody molecule which are recognized as foreign by the human immune system.

Although genetic engineering can be used to make almost any desired change in an antibody structure, it cannot as yet provide solutions to some of the most pressing problems in antibody generation. For example, a common goal is affinity maturation, the optimization of antibody-antigen interaction. In most cases, the three dimensional structure of the antigen is unknown, which makes rational design of the antibody binding site so as to increase affinity almost impossible. Therefore, the ability to alter antibody genes at will does not necessarily help in affinity maturation. Similarly, genetic engineering does not allow generation of human antibodies, which are desirable for therapeutic purposes, but which cannot, for ethical reasons, be generated by immunizing volunteers. Problems such as these demand a new approach to antibody generation.

A promising approach relies on searching for antibodies having the desired properties within a large collection (library) of variants. Methods for generating such antibody libraries, and searching through them, have recently been developed (see, for example, (5) and (6) and references therein). This approach is attractive because:
· It relies on strong binding of the antibody to a target antigen, and is therefore well-suited to affinity maturation.
· Antibodies which are not available by immunization (for example, human antibodies) can be accessed.

Currently, the most efficient approach to antibody affinity maturation uses the following technique. Antibody genes from a suitable source (such as, for example, human peripheral blood lymphocytes, or human bone marrow) are cloned in bacteriophage in such a way that antibody fragments are displayed as fusion proteins on the surface of the phage (7). The phages are produced from bacterial host cells, generating a "phage library" in which every phage contains the genetic information for the antibody variant displayed on its surface. This library can be searched for antibodies which bind to the target antigen.

Locating the highest affinity antibodies within such a library is typically performed by screening: a physical process in which high affinity antibodies are separated from others through their ability to bind to immobilized antigen. The screening process involves immobilizing the target substance on a solid support and performing affinity chromatography or "panning" (8) of the phage library. Those antibodies with high affinity for the immobilized antigen are thereby enriched, and their numbers can be increased by propagation of the specifically eluted phage in bacterial host cells.

Initial results show that antibodies with moderate target affinity can be generated in this way. Higher affinities can, in principle, be obtained by performing random mutagenesis on the antibodies isolated in the first screening, and repeating the process one or more times. For example, random mutagenesis of antibody-encoding genes (9), and random shuffling of the genes encoding the component chains of the antibody (10) have been used to generate new antibodies with increased affinity or altered specificity which have been located within the mutant libraries by screening.

Although a useful tool for antibody affinity maturation, library screening suffers from two main disadvantages. First, the probability of finding a high affinity antibody is related to the size of the library. Due to technical limitations associated with the efficiency with which bacterial cells can be transformed by plasmid DNA, libraries rarely contain more than 10⁸ members. This is not large enough to contain high affinity antibodies routinely. Second, phage isolated by screening must be used to re-infect bacteria if the system is to be run over multiple rounds. The overall process is therefore discontinuous, and as the physical separation and re-infection steps are time-consuming and labour intensive, the method is not well suited to multiple rounds of screening.

These disadvantages are overcome by the present invention, which provides for an artificial method of antibody optimization based on biological selection rather than screening. The invention provides for a library of antibodies, or other ligand or receptor binding oligo- or polypeptides, to be displayed on the surface of phage. The invention requires that the phage are rendered non-infectious by modification of a minor coat protein required for infectivity. Phage which display oligo- or polypeptides with high affinity for a target ligand or receptor are selected from a library by conferring on them the ability to be propagated. The invention provides that phage displaying oligo- or polypeptides with lower target affinity are not propagated. Infectivity is conferred by a substance comprising the target ligand or receptor linked to a portion of the phage coat protein which is required for infectivity.

The main advantage over existing methods offered by the present invention is that it can be carried out in a continuous fashion. In this regard, it mimics the system of clonal selection used by the immune system in antibody optimization. The present invention provides a system which is well-suited to affinity maturation of antibodies in multiple rounds of mutation and selection. Even at a single step, the present invention provides an enormous simplification over existing methods since it provides for propagation of only binding variants, thus obviating the requirement for any chromatography or "panning" step. Extremely large libraries can, in principle, be screened. Furthermore, single binding events are detected, giving the method which is the subject of the present invention a very high sensitivity. Finally, the invention is not restricted to antibody selection; it applies equally to any oligo- or polypeptide which interacts with a target ligand or receptor.

### Detailed Description of the Invention

The present invention allows for the selection, from a library of specially modified phage displaying on their surface ligand binding proteins, of those which display ligand binding proteins which bind most tightly to a target ligand or receptor. The invention uses several components, which are described in more detail below.

Accordingly, the present invention relates to a method for selecting a gene by artificially conferring on it a preferential ability to be replicated, by using an infectious replicable genetic package (RGP) to couple the replicability of the gene to the interaction between the peptide or protein encoded by the gene and a particular target ligand or receptor. In this context, the term infectious replicable genetic package refers to an entity, such as a virus, or bacteriophage which can be replicated following infection of a suitable host cell. For example, the RGP may be one of the class of viruses known as bacteriophage, which infect bacterial cells. The term target ligand refers to any substance which is able to interact with the peptide or protein encoded by the gene to be selected.

In a further embodiment, the present invention relates to a method for selecting genes encoding ligand or receptor binding peptides or proteins (LBPs). In this context, the term LBP refers to any oligo- or polypeptide, or protein which is able to bind, to a greater or lesser extent, a target ligand or receptor. Particularly preferred is a method comprising the following steps (see Figure):
First, an infectious RGP is modified so as to remove its ability to infect a host cell, such modification being so as to allow infectivity to be restored as described below. The resulting RGP is thus non-infectious. For example, in the case where the RGP is a bacteriophage, this is most readily achieved by modifying, so as to render defective, all copies of the coat protein which is essential in enabling the phage to infect a bacterial host cell.
Second, a set of DNA sequences encoding a genetically diverse collection of LBPs is inserted into the genome of the non-infectious RGP, using well-established techniques, in such a way that the LBPs are displayed on its surface. This may, for example, include the use of an expression phagemid and a separate helper virus or phage. By way of example, the LBP may be an antibody single chain Fv fragment. In this case, the target ligand is an antigen to which the fragment should bind. The LBPs may be heterologous, i.e. belong to a single family of substances, such as the antibodies, but possess different amino acid sequences, as defined by the sequences of the DNA which encodes them. Typically, the LBPs, and the DNA sequences which encode them constitute a large collection or library of related, but genetically diverse substances.
Third, the genetic material is expressed in a recombinant host organism, thereby producing non-infectious RGPs displaying LBPs on their surface. In the case where the RGP is a bacteriophage, display of the LBPs can be achieved using existing protocols. For example, single chain Fv (scFv) or Fab antibody fragments can be displayed as fusions with either the gene III or gene VIII protein of the male-specific bacteriophages fd, f1, or M13 as disclosed by Winter et al. (11).
Fourth, host cells, non-infectious RGPs, and a substance referred to as an infectivity mediating complex (IMC) are combined. In this context, the IMC comprises a first portion which selectively interacts with one or more LBPs, and a second portion which confers on the corresponding non-infectious RGP the ability to infect host cells. For example, it has been shown that the infectivity of the filamentous bacteriophage fd-tet can be banished by removing 169 amino acids from the N-terminal portion of the gene III protein (12). This polypeptide is referred to hereafter as the infectivity poypeptide. An IMC may then consist of this infectivity polypeptide covalently linked to a target ligand or receptor. The complex may comprise at least two domains if the ligand is peptidic. Alternatively, the infectivity polypeptide may be covalently joined to the ligand or receptor using any suitable chemical cross-linking procedure (13).
Fifth, the host cells are propagated in liquid culture under conditions which allow equilibrium to be established between the LBPs displayed on the surface of the RGPs and the IMC. Those RGPs which become tightly associated with the IMC by interaction between a displayed LBP and the ligand thereby acquire infectivity. Within the library of LBPs there will be a range of binding affinities for the ligand portion of the IMC. The system can be tuned to select for LBPs having a desired affinity constant by adjusting the concentration of the IMC in the culture medium. At high IMC concentrations, RGPs displaying LBPs with only moderate affinity for the target are infectious. As the concentration of the IMC is lowered, infectivity becomes restricted to those RGPs presenting LBPs with higher target affinity.
Only those RGPs which infect host cells can be replicated. These RGPs are secreted by the host cell, and are able to enter the cycle again. The medium becomes enriched in RGPs which display ligand-binding proteins with high affinity for the target ligand. Under suitable conditions, the system selects for LBPs which bind the ligand most strongly.
In a further step,the gene(s) encoding the LBP(s) displayed on the surface of the RGP(s) may be isolated. This is achieved by standard methods, for example, by PCR of the RGP genome using appropriate primers. The gene(s) thus isolated may be modified in any desired way, re-inserted into the RGP genome, and re-subjected to the selection process.

In a preferred embodiment of the present invention, the RGP described above is a filamentous phage (14). Filamentous phage offer the advantage m the present system that methods for the display of proteins on their surface have been developed (14). Particularly preferred are the filamentous phages of class I (such as fd, M13, f1, If1, Ike, ZJ/2, or Ff), and class II (such as Xf, Pf1, or Pf3).

In a preferred embodiment, the set of DNA sequences encoding a genetically diverse collection of LBPs replaces part of a gene encoding a surface protein, which is essential for binding to, and infection of, a host cell. This partial replacement must not compromise assembly of the phage particle. Particularly preferred is the case in which the surface protein gene described above is gene III of the filamentous bacteriophages. The set of DNA sequences may, for example, replace 507 nucleotides encoding 169 amino acids at the N-terminus of the gene III protein, which are essential for binding to F-pili of host cells.

In a preferred embodiment, the ligand-binding proteins should be immunoglobulins, or members of the immunoglobulin super-family, or any fragments thereof. In this context, the term immunoglobulins includes members of the classes IgA, IgD, IgE, IgG, and IgM. The term immunoglobulin super-family refers to all proteins which share certain structural characteristics with the immunoglobulins, including, for example, the T-cell receptor, or any of the molecules CD2, CD4, CD8 etc. Also included are fragments which can be generated from these molecules, such as Fv (a complex of the two variable regions of the molecule), single chain Fv (an Fv complex in which the component chains are joined by a linker molecule), Fab, or F(ab')2 (15).

In a preferred embodiment, the IMC is a single polypeptide chain comprising a first portion which, when closely associated with non-infectious RGP(s), confers on it/them the ability to infect host cells, and a second portion which binds, to a greater or lesser extent, to one or more LBPs. In this context, the second portion of the polypeptide may be a small peptide ligand, or it may be a receptor molecule. Also preferred is the case in which the IMC comprises a polypeptide which, when closely associated with non-infectious RGP(s), confers on it/them the ability to infect host cells, covalently linked to a non-peptidic ligand for one or more LBPs. In either case, the first portion of the IMC may, for example, comprise the N-terminal section of the gene III protein essential for infectivity. Alternatively, the IMC may incorporate any other protein which has the binding characteristics of the gene III protein. In this context, the term binding characteristics refers to the ability of a protein to interact with the F-pili of host bacterial cells. Specifically included are proteins encoded by a DNA sequence capable of hybridizing with gene III. In this context, the term hybridization refers preferentially to conventional hybridization conditions. Particularly preferred are stringent hybridization conditions.

In a preferred embodiment, the present invention also allows for subjecting the set of DNA sequences encoding a genetically diverse collection of LBPs to random or site-specific mutagenesis. In this context, random mutagenesis refers to the introduction of changes in the identity of the bases making up the set of DNA sequences, at random positions throughout its length. In contrast, the term site-specific mutagenesis refers to defined changes which are made at precise points in the sequence.
It is envisaged that mutagenesis may be performed by any one of a number of methods. For example, both random and site-specific mutagenesis may be effected by replicating the vector which contains the set of DNA sequences to be mutated using an oligonucleotide "cassette" according to well-established methods (16). Furthermore, random mutagenesis may be performed by any one of several additional methods. For example, mutator strains of bacteria can be used as host cells. Commonly used mutators are the mutD, mutH, mutL, mutS, or mutT strains of Escherichia coli, which show deficient repair mechanisms, and therefore increased mutation rates.
Alternatively, random mutagenesis can be achieved by adding during host cell propagation a chemical mutagen, such as formaldehyde, hydroxylamine, methoxyamine, nitrous acid, bisulfite, hydrazine, N-ethyl-N-nitrosourea, or N-methyl-N'-nitro-N-nitrosoguanidine. Other mutagens, such as those listed in (17) may also be used.
Random mutagenesis may also be achieved by subjecting the set of DNA sequences to an error-prone polymerase chain reaction, as described for example in (18).

In a preferred embodiment, random mutagenesis is achieved by any combination of DNA oligonucleotide cassette-based mutagenesis, the use of mutator strains of bacteria, addition of a mutagen during the propagation of host cells, and subjecting said set of DNA sequences to an error-prone polymerase chain reaction.

In a further embodiment, the present invention relates to a kit for the selection of genes encoding LBPs comprising a specially designed vector, and an infectivity mediating complex precursor. The vector may be any one suitable for phage production, and must additionally contain a cloning site at which DNA can be readily inserted. In this context, the term cloning site refers to a region of the vector in which there is at least one restriction enzyme cleavage site, which can conveniently be used to insert a foreign DNA sequence. In a preferred embodiment, there are multiple restriction enzyme cleavage sites at this position. By way of example, the vector fd-tet (12) can, with appropriate modifications, be used according to the present invention.

The vector also displays the following features. It must be capable of being packaged as an infectious RGP, and contain a cloning site enabling the introduction of a set of DNA sequences encoding a genetically diverse collection of LBPs in such a way that the LBPs are displayed at the surface of the RGP when the vector is packaged.

Furthermore, the RGP must include a modification which removes its ability to infect a host cell, although the modification must be so as to allow infectivity to be restored by interaction between an LBP displayed on the surface of the RGP and the IMC.

The term IMC precursor refers to substance which can be used to prepare a complex comprising a ligand which is capable of binding to one or more LBPs, and a polypeptide which, when associated with an RGP, confers on it the ability to infect a host cell. For example, the IMC precursor may comprise a polypeptide which, when associated with an RGP, can confer on it the ability to infect a host cell, and which is, or can be, derivatized in such a way as to allow covalent attachment of a ligand to which one or more of LBPs is able to bind. In this context, derivatization refers to any chemical or biochemical modification which enables the formation of a covalent bond to a second entity. By way of example, any suitable cross-linking reagent can be used, such as N-succinimidyl 3-(2-pyridyldithio)propionate (SPDP), succinimidyl 4-(N-maleimidomethyl) cyclohexane-1-carboxylate (SMCC), m-maleimidobenzoyl-N-hydroxysuccinimide ester (MBS), N-succinimidyl (4-iodoacetyl) aminobenzoate (SIAB), or 1-ethyl-3-(3-dimethylaminopropyl) carbodiimide (EDC) (13).
Alternatively, the IMC precursor may comprise a vector incorporating three DNA sequences: a first DNA sequence encoding a portion of an infectivity polypeptide which, when associated with an RGP can confer on it the ability to infect a host cell; optionally, a second DNA sequence encoding a linker; and an adjacent cloning site which allows introduction of a third DNA sequence encoding a ligand to which one or more LBPs is able to bind.

In a preferred embodiment of the present invention, the specially designed vector can be packaged, and replicated as a filamentous bacteriophage. Particularly preferred are the filamentous bacteriophage of class I (such as M13, f1, If1, Ike, ZJ/2, or Ff) and class II (such as Xf, Pf1, or Pf3).

In a preferred embodiment, the cloning site replaces part, or all of a gene encoding a surface protein which is required by the RGP for binding to, and infection of, a host cell. For eaxmple, this may be gene III of the filamentous bacteriophages and the cloning site may replace the N-terminal 169 amino acids of the gene.

Further embodiments of the present invention are characterised in the claims.

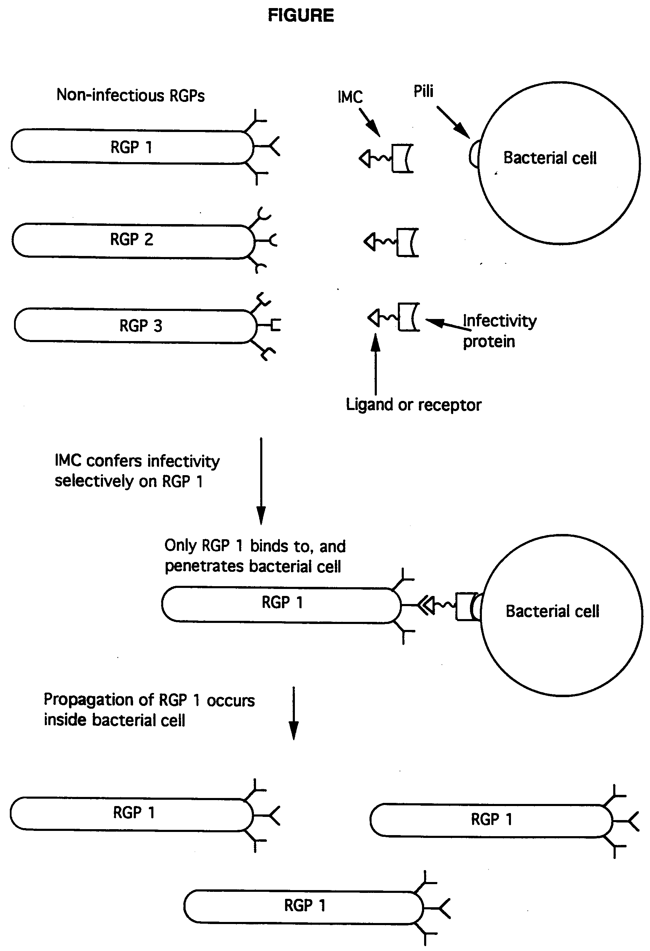

The figure shows:
**Figure:** Selective infection by one of three replicable genetic packages (RGPs), each of which displays a different type of ligand binding protein (LBP). The infectivity mediating complex (IMC) comprises an infectivity protein linked to a ligand or receptor to which the LBP displayed on the surface of RGP 1 binds. Selective interaction between RGP 1 and the IMC results in infection of bacterial host cells by RGP 1, only. RGP 1 is propagated, RGPs 2 & 3 are not.

The example illustrates the invention:

### Example 1: Selective infection of bacterial cells by phage displaying an antitryptophan synthase antibody, conferred by an infectivity mediating complex comprising a portion of tryptophan synthase, linked to a portion of gene III protein.

In the following description, all molecular biology experiments are performed according to standard protocols (16).

### 1. Construction of the infectivity mediating complex (IMC).

Long and short versions of gene III are amplified and cloned. In each case, PCR primer pairs are used which encode BspHI and BspEI restriction sites at the N-terminus and C-terminus respectively of the resulting cloned gene. Using the PCR primer pair 
AAT CAT GAA AAA ATT ATT ATT CGC AA and AAA AGC TTA GTG
ATG GTG ATG GTG ATG TCC GGA ACC GGA GCC,
the N-terminal domain of the gene III protein of bacteriophage M13 is amplified and cloned from the vector M13mp18 (19). In this case, the first 256 amino acids of the gene III protein are encoded, including the long, glycine-rich linker, together with an 18 amino acid signal sequence at the N-terminal end. This is advantageous when separately folding domains are to be fused to the gene III protein. Alternatively, the PCR primer pair 
AAT CAT GAA AAA ATT ATT ATT CGC AA and AAA AGC TTA TCC GGA CGG
AGC ATT GAC AGG AGG TTG
is used to amplify and clone the N-terminal region. In this case, only the first 203 amino acids of the gene III protein are encoded, and the long, glycine-rich region is not amplified. This is desirable to avoid excessive proteolysis, such as when it is necessary to make a fusion to a peptide. Each PCR product is cut with BspHI and BspEI.

An expression vector suitable for secreting proteins is prepared. In this case, a derivative of pASK40 is used (20), which contains a BspHI site overlapping the ATG start codon of the signal sequence, as well as a BspEI site. The two PCR products are cloned into the pASK40 derivative, giving the two vectors pCK101 and pCK102.

The two ligands tested in this case are fragments of E. coli tryptophan synthase (21). The C-terminal fragment of the β-subunit of tryptophan synthase is amplified from the E. coli genome by PCR using the primer pair 
AAT CAT GAC CGA TGA TGA
AGC CC and AAA AGC TTA GTG ATG GTG ATG GTG ATG GAT TTC CCC TCG
TGC TTT.
The complete β-subunit of tryptophan synthase is similarly amplified using the primer pair 
AAT CAT GAC AAC ATT ACT TAA CCCC and AAA AGC
TTA GTG ATG GTG ATG GTG ATG GAT TTC CCC TCG TGC TTT.
In each case, the primers also encode a C-terminal stretch of six histidines, which are included to facilitate purification of the fusion proteins by immobilised metal ion affinity chromatography (22). The two PCR products, and the two plasmids pCK101 and pCK102 are cut with BspEI and BglI. Ligation of each PCR product, vector, and the connecting linker oligonucleotide having the sequence CCGG ACC GGG/CAT GCC CGGT,
generates four product vectors.

The resulting four vectors pCK103, pCK104, pCK105, and pCK106 contain DNA encoding the fusion proteins:
pCK103: the first 169 amino acids of gene III connected to the whole of E. coli tryptophan synthase β-subunit,
pCK104: the first 169 amino acids of gene III connected to the C-terminal 71 amino acids of E. coli tryptophan synthase β-subunit,
pCK105: the first 134 amino acids of gene III connected to the whole of E. coli tryptophan synthase β-subunit,
pCK106: the first 134 amino acids of gene III connected to the C-terminal 71 amino acids of E. coli tryptophan synthase β-subunit.

The fusion proteins so produced are isolated from either of the E. coli strains W3110 or JM83 using Ni(II)-NTA chromatography according to standard methods (22).

### 2. Construction of the RGP.

In this example, a phagemid is used. The tryptophan synthase proteins described in the previous section are recognised by the antibody 93-6 (21) which has been expressed as an Fab fragment using a standard periplasmic expression vector (23). In this vector, the light chain is fused to a shortened version of gene III, containing residues 214 to 424, following the work of Bass et al. (24). E. coli strain JM101 harbouring this plasmid is co-infected with the phage fKN-16 (12) which carries a 507 nucleotide deletion in gene III. Since this phage is non-infective, an extremely high phage titre needs to be used to obtain successful infection with this helper phage.

Next, bacteria are grown using standard methods (25) and phage are harvested as described. The phage comprise a mixture of packaged helper phage and packaged phagemid, each containing antibody-gene III fusion proteins in their coats. The two types of phage are easily discriminated, since the helper phage genome carries a gene encoding tetracyclin resistance, while the phagemid is ampicillin resistant. Phage are enriched by the usual PEG precipitation procedure (25).

### 3. Selective Infection.

Phage and the IMC are mixed at about 1:1 stoichiometry and added to logarithmically growing E. coli JM101 cells. Both tetracyclin and ampicillin resistant colonies are obtained. Upon addition of tryptophan synthase, which interferes with binding of the β subunit to the fusion protein displayed on the surface of the phage, the number of colonies is drastically reduced. Furthermore, only few background clones are obtained if an irrelevant Fab fragment is added, for example, one directed against phosphorylcholine (26) expressed in the same phagemid vector.

The following example provides an improved demonstration of the method.

### Example 2: Application of the Method to Selection Between Two Antibody Fragments Recognizing Different Target Substances.

### A. The Tryptophan Synthase β-Subunit/H936 Anti-Tryptophan Synthase System

### (i) Construction of the Parent Cloning Vector pCK-XBD.

The parent vector for all subsequent steps, pCK-XBD, was constructed from pASK29-L220 (27) by insertion of the self-complementary oligonucleotide CTA GAT AAG GAA AAA TCA TGA TTT TTC CTT AT
into the unique XbaI site of the vector, thereby introducing a unique BspHI site. The start codon included in this BspHI site is ideally positioned six nucleotides downstream of the Shine-Dalgarno sequence, allowing for direct expression cloning of PCR products.

### (ii) Construction of the Vectors pCK-IPM and pCK-IPS Encoding-Infectivity Polypeptides Bearing, a Hexa-histidine Tail.

PCR amplification of the N-terminal portion of gene III from the vector M13mp18 (19) was carried out using the primer pair 
AAT CAT GAA AAA ATT
ATT ATT CGC AA and AAA AGC TTA GTG ATG GTC ATG GTG ATG TCC
GGA ACC GGA GCC.
The resulting DNA was digested with BspHI and HindIII, purified, and ligated into the vector fragment of plasmid pCK-XBD which had been digested with the same enzymes. The resulting plasmid pCK-IPM expresses the polypeptide IPM which comprises the N-terminal 256 amino acids of the mature protein III linked to a hexa-histidine tail.

Similarly, the plasmid pCK-IPS was prepared by PCR amplification of gene III from the vector M13mp18 using the primer pair 
AAT CAT GAA AAA ATT
ATT ATT CGC AA and AAA AGC TTA GTG ATG GTG ATG GTG ATG TCC
GGA CGG AGC ATT GAC AGG AGG TTG,
digestion of the resulting DNA with BspHI and HindIII, purification of the fragment, and ligation into the linearized vector derived from plasmid pCK-XBD by digestion with the same restriction enzymes. The resulting plasmid pCK-IPS expresses the polypeptide IPS, which comprises the N-terminal 217 amino acids of the mature protein III linked to the oligopeptide Pro-Ser-Gly-His-His-His-His-His-His.

### (iii) Construction of Vectors Encoding Infectivity Polypeptide-Tryptophan Synthase β-Subunit Fusion Proteins.

The two target ligands of interest in this case are the C-terminal fragment of the β-subunit of tryptophan synthase from E. coli, and the complete β-subunit of tryptophan synthase (21). The encoding DNA was amplified by PCR using the primer pairs 
AAT CAT GAC CGA TGA TGA AGC CC and AAA AGC TTA GTG
ATG GTG ATG GTG ATG GAT TTC CCC TCG TGC TTT
(for amplifying the C-terminal fragment of the β-subunit - TSS) and 
AAT CAT GAC AAC ATT ACT
TAA CCCC and AAA AGC TTA GTG ATG GTG ATG GTG ATG GAT TTC CCC
TCG TGC TTT
(for amplifying the entire subunit - TSB).

The TSS PCR product was digested with BspHI and HindIII. The plasmids pCK-IPM and pCK-IPS were digested with BspEI and HindIII and separately ligated with the TSS fragment and the connecting linker oligonucleotide pair CCG GAC CGG G and CAT GCC CGG T to generate the plasmids pCK-IPMTSS and pCK-IPSTSS respectively.

The TSB PCR product was digested with BspHI. The plasmids pCK-IPM and pCK-IPS were digested with HindIII and the single-stranded overhang was filled in, giving rise to blunt-ended, linearized vectors. These vectors were then digested with BspEI and the vector fragments were separately ligated with the TSB fragment and the connecting linker oligonucleotide pair CCG GAC CGG G and CAT GCC CGG T to generate the plasmids pCK-IPMTSB and pCK-IPSTSB respectively.

The infectivity-mediating complexes (IMCs) (the fusion proteins IPM-TSS, IPS-TSS, IPM-TSB, and IPS-TSB) were expressed in the *E. coli* strain JM83, and purified by Ni(II)-NTA and anion exchange chromatography (22).

### (iv) Preparation of Non-infectious RGPs Displaying a Single-Chain Antibody Recognising Tryptophan Synthase β-Subunit and Carrying a Gene for Kanamycin Resistance.

The single-stranded DNA of phage fd was subjected to oligonucleotide-directed mutagenesis in order to remove a BspHI restriction site (using the oligonucleotide GAG GAC TAA AGA CTT ACG CAT GAG GAA GTT TCC), and to insert sites for the enzymes EcoRI (using TTG ACA GGA GGT TGA GGG AAT TCC AGG TCA GAC GAT T),
BspHI (using CGA ATA ATA ATT TTT TCA TGA TGA AAA TCT CCA AAA AAA A),
StuI and XhoI (using TTT AAT TGT ATC GGT CTC GAG AGG CCT TTA TCA GCT TGC),
providing the vector fCK-VXZ.

To enable insertion of the gene for Kan resistance between gene VIII and gene III, a portion of the gene III promoter was duplicated in fCK-VXZ as follows. The product of PCR amplification of the phage vector fd with the primers 
GCG GGC
CGC AGG CCT CGA GAA ATT CAC CTC GAA AGC AA and CGA ATA ATA
ATT TTT TCA TGA TGA AAA TCT CCA AAA AAA A
was digested with XhoI and BspHI, and ligated into the same sites in the phage fCK-VKZ, providing the phase fCK-I. The gene encoding kanamycin resistance was amplified from the plasmid pACYC177 using the primers 
AGG CCT TAG AAA AAC TCA TCG
AGC and AGG CCT GTT ACA TTG CAC AAG ATA AAA ATA TAT CAT AAT
GAA CAA TAA AAC TGT CT,
and cloned directly into the StuI site of fCK-I, providing the phage-derived vector fCK-KAN.

A single-chain antibody Fv fragment (scFv) having the structure V_{L}-V_{H} was cloned from the hybridoma H936 (21) and inserted into the vector pIG-6 (28). The scFv was amplified from the resulting plasmid by PCR using the primers 
AAA TCA TGA AAA AGA CAG CTA TCG CG and GGT CAC GCT GCG CGT
AAC C.
The PCR product was digested with BspHI and EcoRI and cloned into the fCK-KAN vector fragment which had been digested with the same enzymes giving the phage vector fCK-H936. This phage vector encodes the H936 scFv as an N-terminal fusion of gene III. Phage particles (RGPs) displaying the scFv on their surface were prepared according to standard methods. The phage particles displayed on their surface single-chain antibody fragments that specifically recognized the β-subunit of tryptophan synthase, as demonstrated by ELISA.

### B. The Benzoylampicillin/2H10 Anti-Benzoylampicillin System

### (i) Construction of the Vector pCK-IPMC Encoding an Infectivity Polypeptide Bearing a Pendant Cysteine Residue.

The plasmid pCK-IPM (see Example 2(ii) above) was digested with BspEI and HindIII, and the resulting vector fragment purified, and ligated with the oligonucleotide pair 
CCG GAT GCC CCC ATC ACC ATC ACC ATC ACT AAT
ATT A and AGC TTA ATA TTA GTG ATG GTG ATG GTG ATG GGG GCA T.
The resulting vector, pCK-IPMC, differed from pCK-IPM in that it encoded the amino acids cysteine and proline between the last glycine and the six histidines at the C-terminus of the modified protein III expressed by pCK-IPM. The protein IPMC was obtained by expression of pCK-IPMC in the strain JM83, and purified by Ni(II)-NTA and anion exchange chromatography (22).

### (ii) Construction of an infectivity-mediating complex by chemical coupling of Benzoyl ampicillin to an infectivity polypeptide bearing a pendant cysteine residue.

Ampicillin was coupled to the water-soluble cross-linker m-maleimidobenzoyl-N-hydroxysulphosuccinimide ester, and the resulting complex was conjugated to the pendant cysteine residue of the IPMC protein using standard methods. The resulting infectivity-mediating complex IPMC-BzAmp was freed of excess ampicillin-MBS by gel filtration chromatography.

### (iii) Preparation of Non-Infectious RGPs Displaying a Single-Chain Antibody Recognising Benzoyl Ampicillin and Carrying a Gene for Kanamycin Resistance.

The single-chain antibody Fv fragment having the structure VL-VH of the hybridoma 2H10 (29) was cloned according to standard methods into the pIG-6 vector (28). This vector was digested with EcoRV and EcoRI, and the fragment was cloned into the phage vector fCK-H936 which had been digested with the same enzymes, resulting in replacement of the H935 scFv gene by the corresponding gene for 2H10. The resulting phage vector, fCK-2H10 enabled preparation of phage particles (RGPs) according to standard methods. The phage particles displayed on their surface single-chain antibody fragments that specifically recognized benzoyl ampicillin, as demonstrated by ELISA.

### C. Specific Restoration of Infectivity of Non-Infectious Phage in the Presence of IMCs.

A 1:1 mixture of RGPs displaying the H936 and 2H10 scFvs was prepared and incubated with either IPM-TSS, IPS-TSS, IPM-TSB, IPS-TSB (see A(iii) above) or IPMC-BzAmp (see B(ii) above). After 4 h at 25°C or 12 h at 4°C, the mixture was added to a suspension of logarithmically growing E. coli XL1-blue cells, which were starved by shaking for 1 h in 80 mM NaCl and 10 mM MgSO4 at 37°C. After incubation for 1 h at room temperature, the cells were plated on YT-agar containing 50 µg/mL of kanamycin.

The use of either IPMTSB, IPSTSB, IPMTSS, or IPSTSS fusion proteins as IMCs resulted in infection of the bacteria by fCK-H936. In contrast, when IPMC-BzAmp was used as IMC, fCK-2H10 clones were specifically formed.

In the complete absence of any IMCs or precursors thereof, the phage particles described in A(iv) and B(iii) above were demonstrated to give rise to substantially fewer colonies than were obtained in the presence of the IMCs as described above. The use of either IPMC, IPM, or IPS instead of any of the IMCs resulted in the same number of clones, showing that there is only a low background of non-specific infection events.

### References

1. Skerra, A. & Plückthun, A. *Science*, **240**, (1988), 1038.
2. Better, M., Chang, C. P., Robinson, R. R. & Horwitz, A. H. *Science*, **240**, (1988), 1041.
3. Morrison, S. L. *Ann. Rev. Immunol.* **10**, (1992), 239.
4. Jones, P. T., Dear, P. H., Foote, J., Neuberger, M. S. & Winter, G. *Nature,* **321**, (1986), 522.
5. Huse, W. D., Lakshimi, S., Iverson, S. A., Kang, A. S., Alting-Mees, M., Burton, D. R., Benkovic, S. J. & Lerner, R. A. *Science*, **246**, (1989)1275.
6. Garrard, L. J. & Zhukovsky, E. A. *Current Opinion in Biotechnology,* **3**, (1992), 474-480.
7. McCafferty, J., Griffiths, A. D., Winter, G. & Chiswell, D. J. *Nature*, **348**, (1990), 552.
8. Garrard, L. J., Yang, M., O'Connell, M. P., Kelley, R. F. & Henner, D. J. *Bio/Technology* **9**, (1991), 1373.
9. Gram H., Marconi, L-A. Barbas, C.F., Collet, T.A., Lerner, R. A. & Kang, A. S. *Proc. Nat. Acad. Sci., U.S.A.,* **89**, (1992), 3576.
10. Marks, J. D., Griffiths, A. D., Malmqvist, M., Clackson, T.P., Bye, J.M. & Winter G *Bio/Technology* **10**, (1992), 779-783.
11. Patent application WO 92/01047.
12. Nelson, F. K., Friedman, S. M., &. Smith, G.P. *Virology* **108**, (1981), 338.
13. Means, G. E., & Feeney, R. E. *Bioconjugate Chemistry,* **1**, (1990), 2.
14. Smith, G. P. *Current Opinion in Biotechniology,* **2**, (1991), 668.
15. Plückthun, A. *Immunol. Rev*. **130**, (1992,), 151.
16. "Molecular Cloning, A Laboratory Manual", Sambrook, J., Fritsch, E. F., & Maniatis, T. (eds), 2nd Ed., Cold Spring Harbor Laboratory Press, 1989.
17. Singer, B., & Kusmierek, J. T. *Ann. Rev. Biochem.* **52**, (1982), 655.
18. Leung, D. W., Chen, E, & Goeddel, D. V. *Technique* **1**, (1989), 11.
19. Ebright, R., Dong, Q., & Messing, J. *Gene,* **114**, (1992), 81.
20. Skerra, A., Plückthun, A., & Pfitzinger, **1**. *Bio/Technology*, 9, (1991), 273.
21. Friguet, B., Djavadi-Ohaniance, L., & Goldberg, M. E. *Res. Immunol.* **140**, (1989), 355.
22. Hochuli, E., Bannwarth, W., Döbeli, H., Gentz, R., & Stüber, D. *Bio/Technology,* **6**, (1988), 1321.
23. Plückthun, A., & Skerra, *Methods in Enzymology,***178** (1989), 497.
24. Bass S., Greene, R., & Wells, J.A. *Proteins*, **8**, (1990), 309.
25. "An Introduction to Recombinant DNA Techniques", Hackett, P. B., Fuchs, J. A., & Messing, J.W. (eds), Benjamin/ Cummings, Menlo Park, 1984.
26. Skerra, A., & Plückthun, A. *Science,* **240**, (1988), 1038.
27. Skerra, A., & Plückthun, A. *Protein Engineering,* **4**, (1991), 971-979.
28. Ge, L., Knappik, A., Pack, P., Freund, C, & Plückthun, A. in "Antibody Engineering: A Practical Approach", IRL Press, C. Borrebaeck et al. (Eds), in press.
29. Suckling, C. J., Tedford, C. M., Proctor, G. R., Khalaf, A. I., Bence, L. M., & Stimson, W. H. in "Catalytic Antibodies", Ciba Foundation Symposium **159**, John Wiley & Sons Ltd., 1991, pp. 201-208.

## Claims

1. A method for selecting a gene by artificially conferring on said gene a preferential ability to be replicated, by using an infectious replicable genetic package (RGP) to couple the replicability of the gene to the interaction between the protein encoded by the gene and a particular target ligand.

2. A method for selecting genes encoding ligand or receptor binding peptides or proteins (LBPs) comprising the following steps:
(a) modifying an RGP in such a way as to remove the ability of said RGP to infect a host cell, such modification being so as to allow infectivity to be restored according to (b) and (d) below;
(b) inserting into the genome of said non-infectious RGP a set of DNA sequences encoding a genetically diverse collection of LBPs in such a way that said LBPs are displayed at the surface of said non-infectious RGP;
(c) expressing in a recombinant host organism the genetic material so as to produce non-infectious RGPs displaying said LBPs;
(d) combining host cells, non-infectious RGPs, and a substance referred to as an infectivity mediating complex (IMC), where the IMC selectively interacts with one or more of said LBPs, and confers on said non-infectious RGPs displaying said LBP(s) the ability to infect said host cells;
(e) propagating host cells under conditions in which RGPs becoming associated with said IMC via LBPs displayed on their surface acquire the ability to infect said host cells and be replicated;
(f) isolating from said host cells RGP carrying the gene or genes encoding the desired LBP, and, optionally;
(g) using said RGP again in the same process from (c) above, an additional one or more times;
(h) isolating said genes from said RGP.

3. The method according to claim 2, wherein said RGP is a filamentous bacteriophage.

4. The method according to claim 3, wherein said filamentous bacteriophage is one of the class I phages fd, M13, f1, If1, Ike, ZJ/2, Ff, and the class II phages Xf, Pf1, and Pf3.

5. The method, according to any of claims 2-4, wherein said set of DNA sequences encoding a genetically diverse collection of LBPs replaces part of a gene encoding a surface protein which is required by said RGP for binding to, and infection of, a host cell.

6. The method according to claim 5 wherein said surface protein gene is gene III of the filamentous bacteriophages.

7. The method according to claim 6, wherein the N-terminal 169 amino acids of said gene III are replaced by said set of DNA sequences.

8. The method according to any of claims 2-7, wherein said LBPs are immunoglobulins, or members of the immunoglobulin super-family, or any fragments thereof.

9. The method according to any of claims 2-8, wherein said IMC is a single polypeptide chain comprising a first portion which, when closely associated with said non-infectious RGP(s), confers on it/them the ability to infect said host cells, and a second domain which is a ligand for one or more of said LBPs.

10. The method according to any of claims 2-8, wherein said IMC comprises a non-peptidic ligand for one or more of said LBPs covalently linked to a polypeptide which, when closely associated with said non-infectious RGP(s), confers on it/them the ability to infect said host cells.

11. The method according to either of claims 9 or 10 in which said IMC comprises, at least in part, the N-terminal 169 amino adds of the gene III protein of the filamentous bacteriophages, or is partially encoded by a DNA sequence hybridizing with said gene III, which encodes a peptide having the binding characteristics of the gene III protein.

12. The method according to any of claims 2-11 which additionally comprises the step of subjecting said set of DNA sequences encoding a genetically diverse collection of LBPs to random or site-specific mutagenesis.

13. The method according to claim 12 wherein said random or site-specific mutagenesis is achieved by DNA oligonucleotide cassette-based mutagenesis.

14. The method according to claim 12 wherein said random mutagenesis is achieved by using, as host cells, mutator strains of bacteria.

15. The method according to claim 14 wherein said mutator strains are any one of the mutD, mutH, mutL, mutS, or mutT strains of Escherichia coli.

16. The method according to claim 12 wherein said random mutagenesis is achieved by adding, during the propagation of said host cells, a mutagen.

17. The method according to claim 16, wherein said mutagen is one or more of formaldehyde, hydroxylamine, methoxyamine, nitrous acid, bisulfite, hydrazine, N-ethyl-N-nitrosourea, or N-methyl-N'-nitro-N-nitrosoguanidine.

18. The method according to claim 12 wherein said random mutagenesis is achieved by subjecting said set of DNA sequences to an error-prone polymerase chain reaction.

19. The method according to claim 12 wherein said random mutagenesis is achieved by any combination of DNA oligonucleotide cassette-based mutagenesis, the use of mutator strains of bacteria, addition of a mutagen during the propagation of host cells, and subjecting said set of DNA sequences to an error-prone polymerase chain reaction.

20. A kit for the selection of genes encoding LBPs comprising:
(a) a vector capable of being packaged as an infectious replicable genetic package (RGP), displaying the following features:
(aa) a cloning site which enables the introduction of a set of DNA sequences encoding a genetically diverse collection of LBPs in such a way that said LBPs are displayed at the surface of said RGP when said vector is packaged;
(ab) a modification which removes the ability of said RGP to infect a host cell, such modification being so as to allow infectivity to be restored by interaction between an LBP displayed on the surface of said RGP and a substance which can confer on said RGP the ability to bind to and infect host cells;
and either or both of;
(b) a polypeptide which can be used in the preparation of an IMC, and which comprises a portion of a protein which, when associated with said RGP can confer on it the ability to infect a host cell, and which is, or can be, derivatized in such a way as to allow covalent attachment of a ligand to which one or more of said LBPs is able to bind;
and/or;
(c) a vector displaying the following features:
(ca) a first stretch of DNA encoding a portion of an infectivity polypeptide which, when associated with said RGP, can confer on it the ability to infect a host cell,
(cb) a second stretch of DNA encoding a linker,
(cc) a third stretch of DNA comprising a cloning site which allows introduction of a one or more DNA sequence(s) encoding (a) ligand(s) or receptor(s) to which one or more of said LBPs is able to bind to a greater or lesser extent.

21. The kit according to claim 20, wherein said RGP is a filamentous bacteriophage.

22. The kit according to any of claims 20 or 21, wherein said filamentous bacteriophage is one of the class I phages fd, M13, f1, If1, 1ke, ZJ/Z, Ff, and the class II phages Xf, Pf1, and Pf3.

23. The kit according to any of claims 20-22, wherein said cloning site replaces part of a gene encoding a surface protein which is required by said RGP for binding to, and infection of, a host cell.

24. The kit according to claim 23, wherein said surface protein gene is gene III of the filamentous bacteriophages.

25. The kit according to claim 24, wherein the N-terminal 169 amino acids of said gene III are replaced by said cloning site.

26. The kit according to claim 20, wherein said derivitization comprises reacting said polypeptide with a commonly used cross-linking reagent, such as SPDP, SMCC, MBS,SIAB,or EDC.


SEQUENCE LISTING

(1) GENERAL INFORMATION:

(i) APPLICANT:
(A) NAME: MorphoSys Gesellschaft fuer
Proteinoptimierung mbH
(B) STREET: Frankfurter Ring 193a
(C) CITY: Munich
(E) COUNTRY: Federal Republic of Germany
(F) POSTAL CODE (ZIP): 80804

(ii) TITLE OF INVENTION: A Method for In Vivo Selection of
Ligand-Binding Proteins

(iii) NUMBER OF SEQUENCES: 23

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: EP 93 10 2484.8
(B) FILING DATE: 17-FEB-1993

(2) INFORMATION FOR SEQ ID NO: 1:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 1:

AATCATGAAA AAATTATTAT TCGCAA 26

(2) INFORMATION FOR SEQ ID NO: 2:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 2:

AAAAGCTTAG TGATGGTGAT GGTGATGTCC GGAACCGGAG CC 42

(2) INFORMATION FOR SEQ ID NO: 3:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 3:

AATCATGAAA AAATTATTAT TCGCAA 26

(2) INFORMATION FOR SEQ ID NO: 4:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 4:

AAAAGCTTAT CCGGACGGAG CATTGACAGG AGGTTG 36

(2) INFORMATION FOR SEQ ID NO: 5:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 5:

AATCATGACC GATGATGAAG CCC 23

(2) INFORMATION FOR SEQ ID NO: 6:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 6:

AAAAGCTTAG TGATGGTGAT GGTGATGGAT TTCCCCTCGT GCTTT 45

(2) INFORMATION FOR SEQ ID NO: 7:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 7:

AATCATGACA ACATTACTTA ACCCC 25

(2) INFORMATION FOR SEQ ID NO: 8:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 8:

AAAAGCTTAG TGATGGTGAT GGTGATGGAT TTCCCCTCGT GCTTT 45

(2) INFORMATION FOR SEQ ID NO: 9:

(i) SEQUENCE CHARACTERISTICS:
(A) LENGTH: 10 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 9:

CCGGACCGGG 10

(2) INFORMATION FOR SEQ ID NO: 10:

(i) SEQUENCE CHARACTERISTICS:
(A) LENGTH: 10 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 10:

CATGCCCGGT 10

(2) INFORMATION FOR SEQ ID NO: 11:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 11:

CTAGATAAGG AAAAATCATG ATTTTTCCTT AT 32

(2) INFORMATION FOR SEQ ID NO: 12:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 12:

AAAAGCTTAG TGATGGTGAT GGTGATGTCC GGACGGAGCA TTGACAGGAG GTTG 54

(2) INFORMATION FOR SEQ ID NO: 13:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 13:

GAGGACTAAA GACTTACGCA TGAGGAAGTT TCC 33

(2) INFORMATION FOR SEQ ID NO: 14:

(i) SEQUENCE CHARACTERISTICS:
(A) LENGTH: 37 base pairs
(B) TYPE: nucleic acid
(C) STRANDEDNESS: single

(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 14:

TTGACAGGAG GTTGAGGGAA TTCCAGGTCA GACGATT 37

(2) INFORMATION FOR SEQ ID NO: 15:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 15:

CGAATAATAA TTTTTTCATG ATGAAAATCT CCAAAAAAAA 40

(2) INFORMATION FOR SEQ ID NO: 16:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 16:

TTTAATTGTA TCGGTCTCGA GAGGCCTTTA TCAGCTTGC 39

(2) INFORMATION FOR SEQ ID NO: 17:

(i) SEQUENCE CHARACTERISTICS:
(A) LENGTH: 38 base pairs
(B) TYPE: nucleic acid

(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 17:

GCGGGCCGCA GGCCTCGAGA AATTCACCTC GAAAGCAA 38

(2) INFORMATION FOR SEQ ID NO: 18:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 18:

AGGCCTTAGA AAAACTCATC GAGC 24

(2) INFORMATION FOR SEQ ID NO: 19:

(i) SEQUENCE CHARACTERISTICS:
(A) LENGTH: 56 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 19:

AGGCCTGTTA CATTGCACAA GATAAAAATA TATCATAATG AACAATAAAA CTGTCT 56

(2) INFORMATION FOR SEQ ID NO: 20:

(i) SEQUENCE CHARACTERISTICS:
(A) LENGTH: 26 base pairs

(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 20:

AAATCATGAA AAAGACAGCT ATCGCG 26

(2) INFORMATION FOR SEQ ID NO: 21:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 21:

GGTCACGCTG CGCGTAACC 19

(2) INFORMATION FOR SEQ ID NO: 22:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 22:

CCGGATGCCC CCATCACCAT CACCATCACT AATATTA 37

(2) INFORMATION FOR SEQ ID NO: 23:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 23:

AGCTTAATAT TAGTGATGGT GATGGTGATG GGGGCAT 37